# EUROPEAN PATENT APPLICATION

(11) **EP 1 425 964 A1**
(43) Date of publication of application: **09.06.2004**
(21) Application number: 02758860.7
(22) Date of filing: 20.08.2002
(51) Int. Cl.: A01K 67/027, G01N 33/15, G01N 33/50

(54) **RAT HIGHLY SENSITIVE TO CARCINOGEN**

(30) Priority: 23.08.2001 JP 2001253241
(71) Applicant: Japan Science and Technology Agency, Kawaguchi-shi, Saitama 332-0012 (JP)
(72) Inventor: SHIRAI, Tomoyuki, Nagoya-shi, Aichi 458-0003 (JP); ASAMOTO, Makoto, Nagoya-shi, Aichi 467-0004 (JP); HOKAIWADO, Naomi; Room 306,, Nagoya-shi, Aichi 466-0 045 (JP)
(74) Representative: Holliday, Louise Caroline
(86) International application number: PCT/JP2002/008373
(87) International publication number: WO 2003/017756

(57) **Abstract**

In order to make it possible to detect a carcinogen highly sensitively andmore easily in a short time, the present invention provides a rat having a high sensitivity to a carcinogen, and a method for detecting carcinogens using the rat, and a method for screening anticancer substances using the rat made to develop a cancer. It has been found that a rat whose normal function in gap junction is inhibited has a high sensitivity to a carcinogen. For the inhibition of normal function in gap junction, part of a connexin gene is made to be deficient and a plasmid vector engineered to carry a gene deficient in connexin function is introduced into a rat to make a transgenic rat. By using the rat of the present invention, it becomes possible to detect a carcinogen highly sensitively and more easily in a short time, and the rat of the present invention made to develop a cancer can be effectively used for the screening of anticancer substances.

SEQUENCE LISTING

<110> JAPAN SCIENCE AND TECHNOLOGY CORPORATION

<120> Carcinogen-hypersensitive rat

<130> A031-43PCT

<140>
<141>

<150> JP P2001-253241
<151> 2001-08-23

<160> 6

<170> PatentIn Ver. 2.1

<210> 1
<211> 1485
<212> DNA
<213> Rattus norvegicus

<220>
<221> CDS
<222> (32)..(883)

<400> 1
cgcagtgcca gggaggtgtg aatgaggcag g atg aac tgg aca ggt cta tac 52
Met Asn Trp Thr Gly Leu Tyr
1               5

acc ttg ctc agt ggc gtg aat cgg cat tct aca gcc att ggc cga gta 100
Thr Leu Leu Ser Gly Val Asn Arg His Ser Thr Ala Ile Gly Arg Val
        10                  15                  20

tgg ctg tcc gtc atc ttt atc ttc aga atc atg gtg ctg gtg gtg gct 148

Trp Leu Ser Val Ile Phe Ile Phe Arg Ile Met Val Leu Val Val Ala
    25                  30                  35

gca gag agc gtg tgg ggt gat gag aag tct tct ttc atc tgt aac acc 196
Ala Glu Ser Val Trp Gly Asp Glu Lys Ser Ser Phe Ile Cys Asn Thr
40                  45                  50                  55

ctc cag ccg ggc tgt aac agc gtc tgc tat gac cat ttt ttc ccc atc 244
Leu Gln Pro Gly Cys Asn Ser Val Cys Tyr Asp His Phe Phe Pro Ile
                60                  65                  70

tcc cat gtg cgc ctg tgg tcc ctg caa ctc atc ttg gtt tcc acc cca 292
Ser His Val Arg Leu Trp Ser Leu Gln Leu Ile Leu Val Ser Thr Pro
            75                  80                  85

gct ctc ctc gtg gca atg cac gtg gct cac caa caa cac ata gaa aag 340
Ala Leu Leu Val Ala Met His Val Ala His Gln Gln His Ile Glu Lys
        90                  95                  100

aaa atg cta cgg ctt gag ggg cac ggg gac ccc ctt cac ctg gaa gag 388
Lys Met Leu Arg Leu Glu Gly His Gly Asp Pro Leu His Leu Glu Glu
    105                 110                 115

gta aag agg cac aag gtg cac atc tca ggg aca ctg tgg tgg acc tat 436
Val Lys Arg His Lys Val His Ile Ser Gly Thr Leu Trp Trp Thr Tyr
120                 125                 130                 135

gtc atc agt gtg gtg ttc cgg ctg ctg ttt gag gct gtc ttc atg tat 484
Val Ile Ser Val Val Phe Arg Leu Leu Phe Glu Ala Val Phe Met Tyr
                140                 145                 150

gtc ttc tat ctg ctc tac ccg ggc tat gcc atg gtg cgg ctg gtc aag 532
Val Phe Tyr Leu Leu Tyr Pro Gly Tyr Ala Met Val Arg Leu Val Lys
            155                 160                 165

tgt gag gcc ttc ccc tgc ccc aac acg gtg gac tgc ttc gtg tcc cgc 580
Cys Glu Ala Phe Pro Cys Pro Asn Thr Val Asp Cys Phe Val Ser Arg

        170                 175                 180

ccc act gag aaa acc gtc ttc act gtc ttt atg ctc gcc gcc tcc ggc 628
Pro Thr Glu Lys Thr Val Phe Thr Val Phe Met Leu Ala Ala Ser Gly
    185                 190                 195

atc tgc att atc ctc aac gtg gcg gag gtg gtg tac ctc atc atc cgg 676
Ile Cys Ile Ile Leu Asn Val Ala Glu Val Val Tyr Leu Ile Ile Arg
200                 205                 210                 215

gcc tgt gcc cgc cgt gct cag cgc cgc tcc aat ccg ccc tcc cgc aag 724
Ala Cys Ala Arg Arg Ala Gln Arg Arg Ser Asn Pro Pro Ser Arg Lys
                220                 225                 230

ggc tcg ggc ttc ggc cac cgc ctc tca cct gaa tac aag cag aat gag 772
Gly Ser Gly Phe Gly His Arg Leu Ser Pro Glu Tyr Lys Gln Asn Glu
            235                 240                 245

atc aac aag ctg ctg agc gag cag gat ggc tct ctg aaa gac ata ctg 820
Ile Asn Lys Leu Leu Ser Glu Gln Asp Gly Ser Leu Lys Asp Ile Leu
        250                 255                 260

cgc cgc agt cct ggc act ggg gcc ggg ctg gct gag aag agc gac cga 868
Arg Arg Ser Pro Gly Thr Gly Ala Gly Leu Ala Glu Lys Ser Asp Arg
    265                 270                 275

tgc tca gcc tgc tga tgccgagtac caggcaacct cccatccaac ccctccctca 923
Cys Ser Ala Cys
280

ccccacccag gcctgcccct ccttctccta tgctggtgag caggcctctg cctcctaggg 983

attactccat caaaccttcc ctccctccct actccccttc ctcagagagt cttctgtcaa 1043

agacctggcc ggcttgggag tggggagcca cttctgcacc agggctcaag gttattgagg 1103

gtgtgggcaa ttctttctgc ctataccctt tcctcttccc tctccctgag atgagggatg 1163

agatgttctg aaggtgtttc caattaggaa acgtaatctt aacccccatg ctgtcaggta 1223

ccccactttg ggagtcatgt cagtggggag ggctgtgagc aagcagagtg gaggaggggc 1283

tctgcactgt ggatggagaa gggaggggag cttgccttgc tgcctgctac aaggaaaagg 1343

aggacacatc tagggtgggg gagttctgga gggagaagca ggcagataaa tcagagtggg 1403

ggttggtcag ggctgccccc agtccccagt tcccaaggcc tctctctctg aaaatgttac 1463

acattaaaca ggattttaca gt 1485

<210> 2
<211> 283
<212> PRT
<213> Rattus norvegicus

<400> 2
Met Asn Trp Thr Gly Leu Tyr Thr Leu Leu Ser Gly Val Asn Arg His
1               5                   10                  15
Ser Thr Ala Ile Gly Arg Val Trp Leu Ser Val Ile Phe Ile Phe Arg
            20                  25                  30
Ile Met Val Leu Val Val Ala Ala Glu Ser Val Trp Gly Asp Glu Lys
        35                  40                  45
Ser Ser Phe Ile Cys Asn Thr Leu Gln Pro Gly Cys Asn Ser Val Cys
    50                  55                  60
Tyr Asp His Phe Phe Pro Ile Ser His Val Arg Leu Trp Ser Leu Gln
65                  70                  75                  80
Leu Ile Leu Val Ser Thr Pro Ala Leu Leu Val Ala Met His Val Ala
                85                  90                  95
His Gln Gln His Ile Glu Lys Lys Met Leu Arg Leu Glu Gly His Gly
            100                 105                 110
Asp Pro Leu His Leu Glu Glu Val Lys Arg His Lys Val His Ile Ser
        115                 120                 125
Gly Thr Leu Trp Trp Thr Tyr Val Ile Ser Val Val Phe Arg Leu Leu

    130                 135                 140
Phe Glu Ala Val Phe Met Tyr Val Phe Tyr Leu Leu Tyr Pro Gly Tyr
145                 150                 155                 160
Ala Met Val Arg Leu Val Lys Cys Glu Ala Phe Pro Cys Pro Asn Thr
                165                 170                 175
Val Asp Cys Phe Val Ser Arg Pro Thr Glu Lys Thr Val Phe Thr Val
            180                 185                 190
Phe Met Leu Ala Ala Ser Gly Ile Cys Ile Ile Leu Asn Val Ala Glu
        195                 200                 205
Val Val Tyr Leu Ile Ile Arg Ala Cys Ala Arg Arg Ala Gln Arg Arg
    210                 215                 220
Ser Asn Pro Pro Ser Arg Lys Gly Ser Gly Phe Gly His Arg Leu Ser
225                 230                 235                 240
Pro Glu Tyr Lys Gln Asn Glu Ile Asn Lys Leu Leu Ser Glu Gln Asp
                245                 250                 255
Gly Ser Leu Lys Asp Ile Leu Arg Arg Ser Pro Gly Thr Gly Ala Gly
            260                 265                 270
Leu Ala Glu Lys Ser Asp Arg Cys Ser Ala Cys
        275                 280

<210> 3
<211> 21
<212> DNA
<213> Artificial Sequence

<220>
<223> Description of Artificial Sequence:Insertion
Sequence

<400> 3
catcatcacc atcaccattg a 21

<210> 4
<211> 20

<212> DNA
<213> Artificial Sequence

<220>
<223> Description of Artificial Sequence:P1

<400> 4
aacgtggcgc aggtggtgta                                                    20


<210> 5
<211> 20
<212> DNA
<213> Artificial Sequence

<220>
<223> Description of Artificial Sequence:P2

<400> 5
atggtgatgg tgatgatggc                                                    20


<210> 6
<211> 21
<212> DNA
<213> Artificial Sequence

<220>
<223> Description of Artificial Sequence:P3

<400> 6
gggaaggttt gatggagtaa t                                                  21

## Description

### Technical Field

The present invention relates to a rat having a high sensitivity to a carcinogen, in particular, a rat having a high sensitivity to a carcinogen whose normal function in gap junction is inhibited, a method for producing the rat and use thereof.

### Background Art

Gap junction, a structure wherein intercalated disks connected to plasma membranes of contiguous cells are juxtaposed to each other and thereby junctions of intercellular gaps are apparently adhered, is found in many cells/tissues. It is presumed that gap junction is responsible for intercellular communication wherein an intracellular signal transmitter is delivered to a contiguous cell, and serves to maintain the homeostasis of cell proliferation in multicellular organisms. Cell canceration can be regarded as cell proliferation which deviates from homeostasis in normal cells, and it is thought that inhibition of gap junctional function plays an important role in cell canceration ("Experimental Medicine" Vol. 11, No. 16, 28-33, 1993; Histol. Histopathol. 12, 761-768, 1997).

For example, it is presumed that even though a gene in a certain cell mutates and consequently there is a cell at the early stage of canceration (an initiation cell), if the intercellular communication ability of the cell directed to surrounding normal cells is maintained, the characteristic expression of the initiation cell as a cancerous cell is inhibited, and as a result, the progress of carcinogenesis process is inhibited. In fact, it is known that about 60% of about 300 kinds of carcinogens and cancer promoters inhibit intercellular communication ability in *in vitro* system using cells. Further, decrease of connexin protein expression is observed in carcinogenesis process of various organs of human, rat and mouse, and it is presumed that the abnormality in gap junction is greatly involved in carcinogenesis.

Gap junction is an intercellular channel comprised of a pair of hemichannels formed by each of the contiguous cells, that is, a pair of connexons. The connexon has a structure wherein a bridge is formed by a cylindrical protein which penetrates into an intercalated disk connected to a plasma membrane of a contiguous cell. This channel has a function to deliver substances with molecular weight up to about 1 kD from the inside of a cell to that of a contiguous cell. The connexon is comprised of an assembly of six proteins called connexin. There are many kinds of connexins, and 12 different connexin cDNAs have been cloned so far. Connexins are designated based on the size of proteins they encode, and called, for instance, connexin 26, connexin 32 (Seq. ID No. 2), and connexin 43 (cx26, cx32, and cx43), respectively ("Experimental Medicine" Vol. 11, No. 16, 28-33, 1993).

A mouse deficient in connexin 32 gene, mainly expressed in the liver, has been already produced and reported by a German group (CANCER RESEARCH, 60, 5087-5091, 2000; Carcinogenesis vol. 20, no. 7, 1379-1382, 1999; Current Biology, 7, 713-716, 1997). However, mice are originally less responsive than rats to hepatocarcinogens and hepatocarcinogenesis modifiers.

Meanwhile, there exists glutathione S-transferase placental form (GST-P) as a precancerous hepatic lesion marker in rats, and therefore, a precancerous lesion as an object of analysis can be measured efficiently in a short time. By contrast, there is no precancerous lesion marker for mice, such as GST-P for rats. ES cells of rats have not been established yet and it is impossible to produce a gene-deficient rat.

The object of the present invention is to provide a rat having a high sensitivity to a carcinogen, a method for detecting carcinogens using the rat, and a method for screening anticancer substances using the rat made to develop a cancer in order to make it possible to detect a carcinogen highly sensitively and more easily in a short time.

In order to attain the above-mentioned object, the present inventors have conducted keen study and found that a rat whose normal function in gap junction, in particular, channel function of connexon, is inhibited, has a high sensitivity to a carcinogen, and thus the present invention has been completed. As for the inhibition of channel function of connexon, part of a connexin gene is made to be deficient and a plasmid vector engineered to carry a gene deficient in connexin function is introduced into a rat to make a transgenic rat. By using the rat of the present invention, it becomes possible to detect a carcinogen highly sensitively and more easily in a short time, and the rat of the present invention made to develop a cancer can be effectively used for the screening of anticancer substances.

### Disclosure of the Invention

The present invention relates to a rat highly sensitive to a carcinogenwhose normal function in gap junction is inhibited (claim 1), the rat highly sensitive to a carcinogen according to claim 1, wherein the inhibition of the normal function in gap junction is an inhibition of channel function of connexon (claim 2), the rat highly sensitive to a carcinogen according to claim 2, wherein the inhibition of channel function of connexon is based on a deficiency of connexin function (claim 3), and the rat highly sensitive to a carcinogen according to claim 3, wherein the deficiency of connexin function is based on amutation of connexin 32 (claim 4).

The present invention also relates to a method for producing a rat highly sensitive to a carcinogen comprising the steps of: constructing a plasmid vector engineered to carry mutated rat connexin cDNA downstream of a promoter, and microinjecting the plasmid vector into a fertilized egg and then transplanting the fertilized egg into an oviduct (claim 5), the method for producing a rat highly sensitive to a carcinogen according to claim 5, wherein the promoter is an albumin promoter and the mutated rat connexin cDNA is a cDNA that encodes an amino acid sequence of rat connexin 32 wherein part of an amino acid is deleted (claim 6), a method for detecting a carcinogen wherein a test substance is administered to the rat highly sensitive to a carcinogen according to any one of claims 1 to 4 (claim 7), a method for screening an anticancer substance wherein a test substance is administered to a rat made to develop a cancer by using the rat highly sensitive to a carcinogen according to any one of claims 1 to 4 (claim 8), and the method for screening an anticancer substance according to claim 8, wherein carcinogenesis of the rat highly sensitive to a carcinogen is caused by the administration of a carcinogen (claim 9).

### Brief Description of Drawings

Fig. 1 is a view showing a plasmid vector Cx32/pGEM-Alb engineered to carry a gene connected to rat connexin 32 cDNA (GeneBank, NM017251) downstream of an albumin promoter, being used in the present invention.
Fig. 2 is a view showing the result of transgene expression confirmed by RT-PCR in total RNA extracted from a transgenic rat (baby rat) produced by the method of the present invention.
Fig. 3 comprises photographs showing the results of fluorescent microscopic observation using immunofluorescent staining of localization of connexin 32 in the livers of a transgenic rat and a wild-type rat of the present invention.
Fig. 4 comprises a photograph of an immunostained precancerous hepatic lesion marker resulted from 20-week observation after administering diethyl nitrosoamine to a transgenic rat and a wild-type rat of the present invention, and a photograph showing measurement results of the number and area of foci per unit area in the lesion.
Fig. 5 is a view showing the result that a transgenic rat exhibits much higher expression in comparison to the expression of CYP1A1 and CYP1A2 in the livers of a transgenic rat (Tg) and a wild-type rat (Wild) of the present invention.

### Best Mode of Carrying Out the Invention

The present invention comprises the production of a rat whose normal function in gap junction is inhibited, in particular, the production of a rat whose channel function of connexon in gap junction is inhibited. So far, ES cells of rats have not been established yet and it is impossible to produce a gene-deficient rat. However, as to connexon, whose channel is formed by connexin molecule hexamers, the function of normal connexin does not express since channel function will be inhibited if any one of the hexamers is a mutant connexin. Therefore, a rat whose normal function in gap junction is inhibited can be produced by producing a transgenic rat having a mutant connexin gene.

There is no particular limitation as to the mutant connexin gene, but mutant connexin 32 gene wherein the function of normal connexin does not express can be advantageously used. Various methods for mutation can be used to produce mutant connexin wherein the function of normal connexin does not express , however, a method using cDNA wherein part of a gene encoding connexin is made to be deficient can be advantageously used. In a transgenic rat mutated by a mutant connexin 32 gene wherein the function of normal connexin does not express, gap junction having normal function is not formed in hepatocytes because there is mutant connexin 32, and the expression of a mutant character by surrounding normal cells through gap junction is not controlled sufficiently in initiated cells. Consequently, a precancerous lesion is in a more advantageous proliferation environment and therefore it expresses a character highly sensitive to a carcinogen.

In order to introduce a mutated rat connexin gene into a rat in the present invention, appropriate methods employed in this field can be used, however, a method comprising the steps of: constructing a plasmid vector engineered to carry mutated rat connexin cDNA downstream of a promoter, and injecting the plasmid vector into a fertilized egg and then transplanting the fertilized egg into an oviduct can be advantageously used. The promoter is not particularly limited, but an albumin promoter is particularly preferable. A transgenic rat can be obtained by the method comprising the following steps: constructing a plasmid vector engineered to carry rat connexin cDNA mutated by, for example, deleting part of an amino acid, downstream of this promoter, and injecting this plasmid vector into a male pronucleus of a rat fertilized egg and then transplanting the fertilized egg into an oviduct of a female being false pregnant.

The rat of the present invention is used to detect carcinogens. That is, the rat highly sensitive to a carcinogen of the present invention is administered with a test substance and then carcinogens are detected. The method for administering a test substance and the method for detecting carcinogens are not particularly limited and methods usually employed in this field are used. Further, in the present invention, it is possible to screen anticancer substances by administering a test substance to an individual made to develop a cancer by using the rat highly sensitive to a carcinogen of the present invention. For carcinogenesis in rat, carcinogens can be used. The method for administering a test substance and the method for detecting anticancer substances are not particularly limited and methods usually employed in this field are used.

The present invention will be described more specifically with examples, but the scope of the present invention is not limited to these examples.

### Example 1 (Construction of transgene)

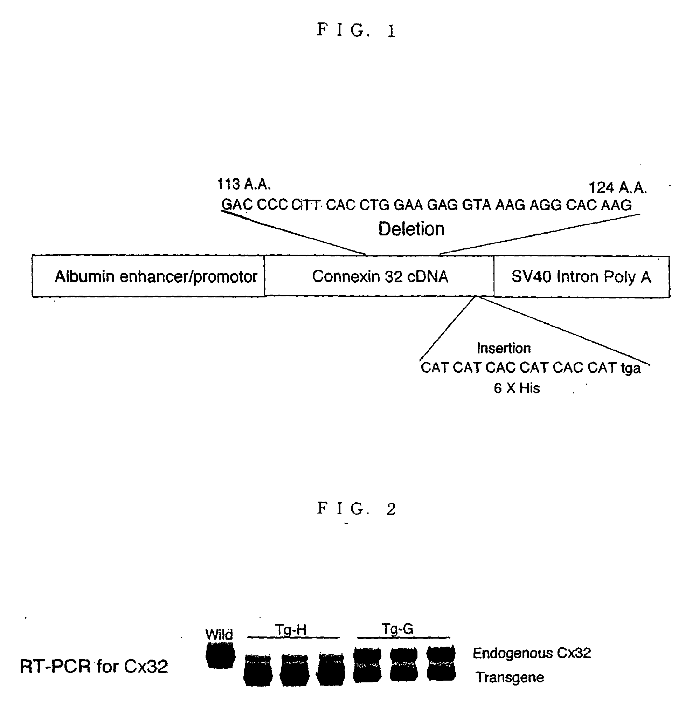

A plasmid vector Cx32/pGEM-Alb engineered to carry a gene connected to rat connexin 32 cDNA (GeneBank, NM017251: Seq. ID No. 1) downstream of an albumin promoter was provided by Dr. Hiroshi Yamasaki (IARC, Lyon, Kwansei Gakuin University, School of Science). The amino acids in the 113^{th} to 124^{th} position of this connexin 32 cDNA were deleted, and further, for a marker to be used after the introduction of this gene, a construct to which six histidines are inserted at the position immediatly before the termination codon (Seq. ID No. 3) was made with the use of ExSite-PCR-Based Site-Directed Mutagenesis Kit (Stratagene) (Fig. 1). This construct was cut out from a plasmid moiety at Apa I and Mlu I, and used as a transgene.

### Example 2 (Production of transgenic rat)

Baby rats were obtained by the method comprising the following steps: microinjecting the above-mentioned transgene into a male pronucleus of a fertilized egg of SD rat, culturing the obtained egg cell, and then transplanting the fertilized egg into an oviduct of a female being false pregnant. DNA was extracted from a tail part of the obtained babies, and the transgene was confirmed by PCR using primer 1 (5'-AACGTGGCGCAGGTGGTGTA-3'; Seq. ID No. 4: P1) and primer 2 (5'-ATGGTGATGGTGATGATGGC-3' ; Seq. ID No. 5: P2) for the transgene, and primer 3 (5'-GGGAAGGTTTGATGGAGTAAT-3'; Seq. ID No. 6: P3) for endogenous connexin 32 (cx32). The primer 2 corresponds to the introduced histidine, and PCR product can be obtained only when both the transgene and the primer 1 exist. In addition, in order to search the expression of the transgene in the liver, total RNA was extracted from the liver and RT-PCR was conducted (Fig. 2). In Fig. 2, Tg-H means a strain of transgenic rats with high expression of the transgene, Tg-G means a strain of transgenic rats with low expression of the transgene, and Wild means wild-type rat (SD rat), respectively. As a result, it was confirmed that the transgene existed in five rats in total, and that four of them transmitted the transgene to the next generation, and that mRNA of the transgene was expressed in the livers of two strains of them. A strain with higher expression was selected from the two strains and used for the following examples. The transgenic rats used for the following examples were obtained by intercrossing male transgenic rats and female wild-type SD rats.

### Example 3 (Immunofluorescent staining of connexin 32)

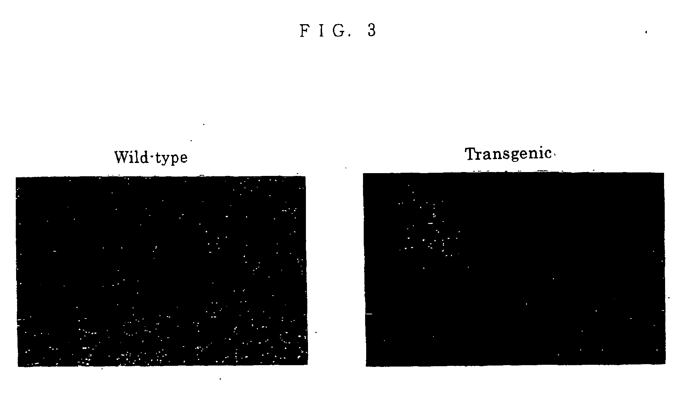

Frozen sections of the livers of the transgenic rats and wild-type rats obtained in Example 3 were fixed with acetone at -20° C for five minutes and followed by air-drying, and then blocked by treating with methanol/H₂O₂. The frozen sections were incubated in the coexistence of anti-connexin 32 rabbit polyclonal antibody (Zymed), followed by immunofluorescent staining with biotinylated anti-rabbit IgG antibody (Vector) and FITC-labeled streptavidin (Vector), then the localization was observed by a fluorescent microscope (AX7C; Olympus). As a result, in the livers of wild-type rats, a number of spotlike localization of connexin 32 was observed on the membranes of contiguous hepatocytes. Meanwhile, no clear localization of connexin 32 was found in the livers of transgenic rats. Thus, it was confirmed that spotlike localization of normal endogenous connexin 32 on the membrane was inhibited by the expression of connexin 32 that had been introduced (Fig. 3).

### Example 4 (Measurement of intercellular communication ability between hepatocytes)

After extracting the liver from a rat, the liver was sliced into pieces about 5 mm thick, and a vertical cut about 1 mm deep was made on the sliced face with a knife. A sufficient amount of 0.05% lucifer yellow was dropped on the cut and the slice was kept still for three minutes, then washed three times with PBS, and embedded in OTC compound (TissuTek, Miles), then frozen. A frozen section of 6 µm thick was made by cryostat and the spread of fluorescence was observed under a fluorescent microscope. From this observation, it has been revealed that in the livers of wild-type rat, the fluorescence taken up through the cells damaged by the knife widely spread to the surrounding hepatocytes by intercellular communication through gap junction. On the other hand, in the livers of transgenic rats, the spread of fluorescence was obviously in low level, and it has been revealed that intercellular communication ability was inhibited.

### Example 5 (Search of sensitivity to hepatocarcinogenesis by diethyl nitrosamine)

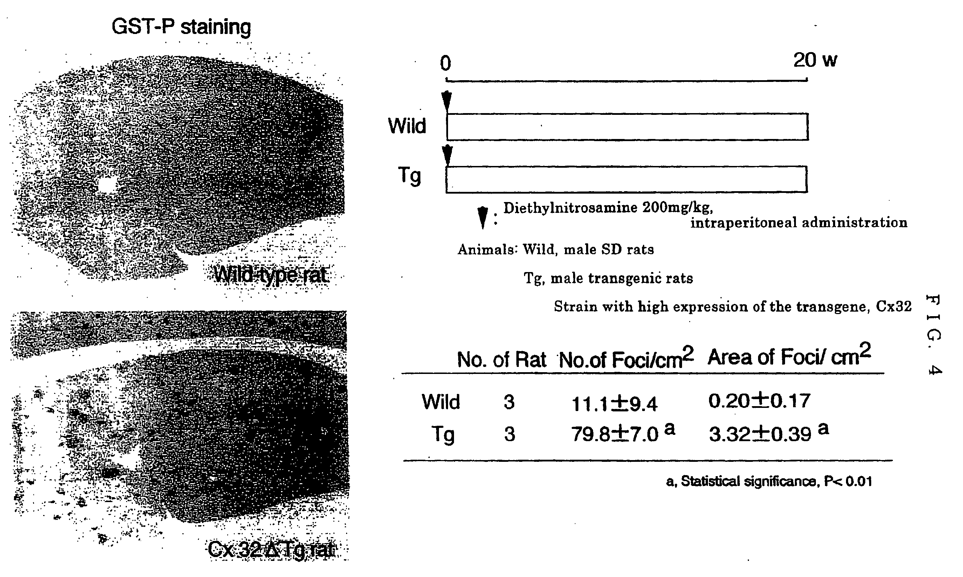

200 mg/kg diethyl nitrosamine was intraperitoneally administered to eight-week-oldmale transgenic rats orwild-type rats, and the rats were observed for 20 weeks after the administration, and then slaughtered and dissected. The livers taken from each of the above-mentioned rats were fixed with acetone and embedded in paraffine, sliced, and then GST-P, a precancerous hepatic lesion marker, was immunostained for visualization (Fig. 4, left). Further, the number and the area of positive foci per unit area in the GST-P-positive foci were measured with an image analyzer (IPAP, Olympus) (Fig. 4, right). As a result of these observations, the number and the area of the GST-P-positive foci, which is precancerous foci, per unit area (1 cm²) in the transgenic rats were remarkably increased in comparison to those in wild-type rats, that is, the number was 11.1 for wild-type rats and 79.8 for the transgenic rats, and the area was 0.2 mm² for the wild-type rats and 3.32 mm² for the transgenic rats. Therefore, it has been found that these transgenic rats exhibit an extremely high sensitivity to diethyl nitrosamine, a hepatocarcinogen.

### Example 6 (Liver-specific gene expression of transgenic rat by cDNA array and RT-PCR)

Total RNA was extracted from the livers of male transgenic rats and wild-type rats by ISOGEN (NIPPON GENE), and the changes in the expression of each gene were searched by using Atlas rat toxicology array (Clontech).

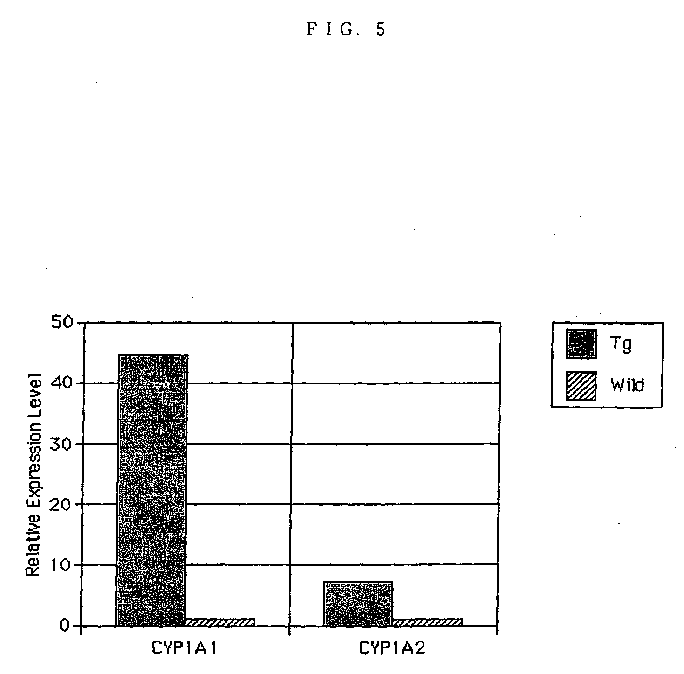

Based on the results, according to the search with cDNA array, genes of drug-metabolizing enzymes such as various molecular species of cytochrome p450 (CYP) and glutathion S-transferase (GST) were fixed as a gene that shows changes in the expression in the transgenic rats compared to that in the wild-type rats. Then, changes in the expression of various molecular species of CYP and GST were examined by quantitative RT-PCR using LightCycler (Loche). According to this examination, the clearest change was that CYP1A1 and CYP1A2 were expressedhighly in the livers of transgenic rats (Fig. 5). These enzymes contribute to metabolic activation of many carcinogens including diethyl nitrosamine, and are thought to be one mechanism of increasing the sensitivity of transgenic rats to hepatocarcinogenesis. Conventionally, inhibition of intercellular communication ability has been considered to be deeply involved in the promotion activity of carcinogenesis, and this experimental result further reveals that inhibition of intercellular communication ability affects the expression of drug-metabolizing enzymes, and makes a situation wherein carcinogens tend to be metabolically activated.

### Industrial Applicability

The rat whose normal function in gap junction is inhibited of the present invention has a high sensitivity to a carcinogen, and makes it possible to detect a carcinogen highly sensitively and more easily in a short time. In addition, the transgenic rat of the present invention made to develop a cancer shows extremely high degree of usefulness, for example, it can be used effectively for the screening of anticancer substances.

## Claims

1. A rat highly sensitive to a carcinogen whose normal function in gap junction is inhibited.

2. The rat highly sensitive to a carcinogen according to claim 1, wherein the inhibition of the normal function in gap junction is an inhibition of channel function of connexon.

3. The rat highly sensitive to a carcinogen according to claim 2, wherein the inhibition of channel function of connexon is based on a deficiency of connexin function.

4. The rat highly sensitive to a carcinogen according to claim 3, wherein the deficiency of connexin function is based on a mutation of connexin 32.

5. A method for producing a rat highly sensitive to a carcinogen comprising the steps of : constructing aplasmidvectorengineered to carry mutated rat connexin cDNA downstream of a promoter, and microinjecting the plasmid vector into a fertilized egg and then transplanting the fertilized egg into an oviduct.

6. The method for producing a rat highly sensitive to a carcinogen according to claim 5, wherein the promoter is an albumin promoter and the mutated rat connexin cDNA is a cDNA that encodes an amino acid sequence of rat connexin 32 wherein part of an amino acid is deleted.

7. A method for detecting a carcinogen wherein a test substance is administered to the rat highly sensitive to a carcinogen according to any one of claims 1 to 4.

8. A method for screening an anticancer substance wherein a test substance is administered to a rat made to develop a cancer by using the rat highly sensitive to a carcinogen according to any one of claims 1 to 4.

9. The method for screening an anticancer substance according to claim 8, wherein carcinogenesis of the rat highly sensitive to a carcinogen is caused by the administration of a carcinogen.
